# EUROPEAN PATENT APPLICATION

(11) **EP 0 895 760 A1**
(43) Date of publication of application: **10.02.1999**
(21) Application number: 98114806.7
(22) Date of filing: 06.08.1998
(51) Int. Cl.: A61F 2/06

(54) **An angioplasty stent, particularly for treating vessels having bifurcations**

(30) Priority: 08.08.1997 IT TO970729
(71) Applicant: SORIN BIOMEDICA CARDIO S.p.A., I-13040 Saluggia, Vercelli (IT)
(72) Inventor: Bartorelli, Antonio, 20121 Milano (IT); Vallana, Franco, 10123 Torino (IT)
(74) Representative: Bosotti, Luciano

(57) **Abstract**

An angioplasty stent comprises a body (1) with a wall (2) having a generally tubular envelope with openings, the stent being expandable, in use, from a radially contracted condition to a radially expanded condition. The configuration with openings is substantially uniform except for at least one opening (3) which has substantially larger dimensions than the remaining openings provided in the wall. The opening (3) extends over only a portion of the angular extent of the body (1) in the respective longitudinal portion of the body (2) of the stent.

Marker means (4) are provided for detecting the angular position of the stent (1) at the implant site.

## Description

The present invention relates to angioplasty stents.

This term defines, in general, a device to be fitted in a lumen (for example, inside a blood vessel), usually by catheterization, and subsequently spread out in situ in order to support the lumen locally. The main purpose of this support is to eliminate a stenotic site present in the location treated by preventing re-establishment of the stenosis.

For a general teaching with regard to vascular stents, reference may usefully be made to the work "Textbook of Interventional Cardiology" by Eric J. Topol., W.B. Saunders Company, 1994 (see, in particular, Section IV of Vol. II, entitled "Coronary stenting") and "Endoluminal Stenting" by Ulrich Sigwart, W.B. Saunders Company, 1996.

A very large number of patent documents is also devoted to the subject as is shown, for example, by US-A-4 776 337, US-A-4 800 882, US-A-4 907 336, US-A-4 886 062, US-A-4 830 003, US-A-4 856 516, US-A-4 768 507, US-A-4 503 569 and EP-A-0 201 466. It should, however, be pointed out that it has already been proposed in the art to use substantially similar structures for spreading out and anchoring vascular grafts in situ; naturally, this possible extension of the field of application should be understood as being included within the scope of the invention.

In spite of the extensive research and experimentation as documented at patent level, only a very small number of operative solutions has up to now been used in practice. This can be attributed to various factors, amongst which the following problems or requirements may be mentioned:
- to ensure that, during its advance towards the site to be treated, the stent can adapt in a sufficiently flexible manner to the path travelled, even in portions having small radii of curvature, and without adversely affecting the ability of the stent to perform an effective supporting action once positioned and released,
- to prevent, or at least limit, the effect of longitudinal shortening which occurs in some stents when they are spread out,
- to offer as extensive as possible a bearing surface to the lumen wall to be supported,
- to avoid giving rise to complex geometry and/or to possible stagnation sites which could cause problems such as coagulation, clotting, etc., and
- to reconcile the requirements set out above with simple and reliable production methods and criteria within the scope of currently-available technology.

The development of angioplasty techniques, particularly in the coronary region, has demonstrated the importance of an ability to intervene effectively on vessels having bifurcations.

When a stent is used for an intervention on a vessel having a bifurcation in the portion subjected to angioplasty, at least two basic problems arise.

In the first place, there is a risk that the wall of the stent extending across the mouth (ostium) of the branch, may block the mouth or at least reduce its net cross-section. This phenomenon is noticed even if the stent wall has a structure with openings (that is, a reticular structure), and is aggravated by the fact that, during the expansion of the stent, the atherosclerotic material may be displaced (by the phenomenon known as "plaque shifting") in the region of the ostium of the branch, restricting the ostium or blocking it completely. In any case, the ramified structure of the stent wall constitutes a preferential site for the development of deposits and/or for the initiation of re-stenosis precisely in the region of the mouth of the branch. The respective branch vessel may be a vessel which is responsible for vascularizing a large muscle region and the blocking of which might cause myocardial infarct, or a vessel responsible for vascularizing a region which is crucial for the correct operation of the heart. An example which applies to all of these is the atrial sinus branch which branches out from the right coronary artery and which is essential for the normal operation of the atrial sinus node which acts as a heart pacemaker.

In the second place, it is important to arrange for the treatment in the region of the bifurcation optionally to affect not only the main vessel but also the vessel which branches out from the bifurcation.

For this purpose, in the stent sold under the trade mark Jostent by Jomed International AB of Helsingborg (Sweden), there is at least one portion characterized by a mesh structure with openings approximately twice the size of the openings in the remaining portion of the stent.

With this particular stent structure, one of the large openings can be positioned in the region of the mouth of the branch vessel in question, which is thus less liable to be blocked. It is then possible by a so-called "culotte" operation, to introduce a further stent into the stent thus positioned by advancing the distal end of the further stent towards the branch vessel through one of the large openings in the first stent. The subsequent expansion of the second stent then offers the possibility of also supporting the wall of the branch vessel, producing a composite Y-shaped support configuration for the walls of the vessels in the region of the bifurcation.

This solution encounters an intrinsic basic difficulty since, precisely owing to the presence of the portion of stent which comprises enlarged openings around the entire periphery of the stent, inadequate support is achieved for the corresponding portion of the vessel wall. This occurs even if the composition and processing of the material used to produce the stent provide a high degree of radial strength distributed uniformly over the entire length of the stent. The problem is in fact of a geometrical nature; where the openings in the stent are large, the points of support of the vessel wall are spaced out and the supporting action is inadequate precisely because the vessel walls and the overlying plaque tend in any case to prolapse into the lumen relative to the support points defined by the elements ("braces") of the stent wall which define the mesh.

The object of the present invention, which has the specific characteristics recited in the following claims, is to solve, in addition to the general problems inherent in the production of angioplasty stents, also the specific problems outlined above with specific reference to the treatment of vessels having bifurcations.

The invention will now be described purely by way of non-limiting example, with reference to the appended drawings, in which:
Figure 1 shows schematically the treatment of vessels having bifurcations by means of stents according to the invention,
Figure 2 is a perspective view of an angioplasty stent formed in accordance with the invention.

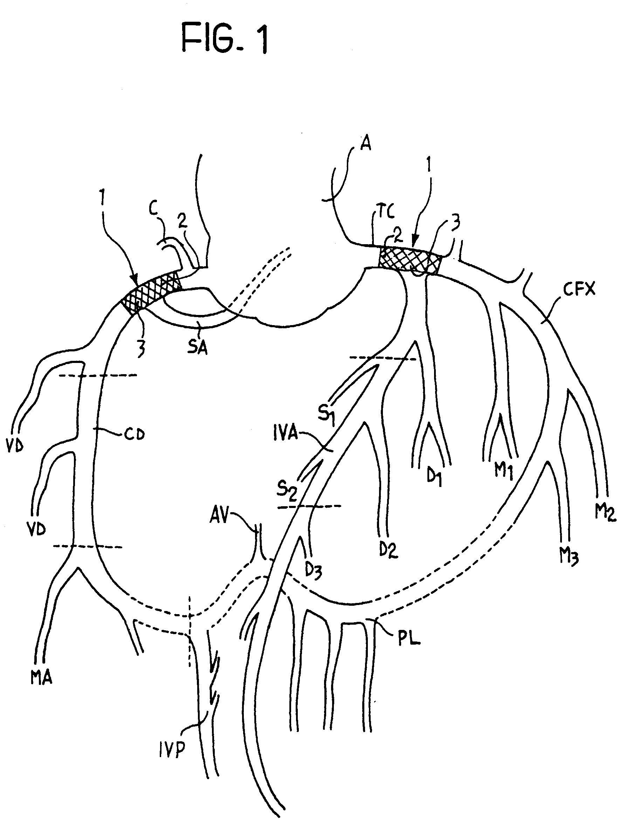

Figure 1 shows the network of coronary vessels in the typical conventional format of the sheets used for coronarography reports.

In particular, the symbols given in Figure 1 identify the following vessels:
- A:: aorta
- CD:: right coronary artery
- C:: conus branch
- SA:: atrial sinus branch
- VD:: right anterior ventricular branches
- MA:: acute marginal branch
- IVP:: posterior interventricular artery
- AV:: atrio-ventricular node branch
- TC:: left common coronary trunk
- IVA:: anterior interventricular artery
- S1, S2:: first and second anterior septal branches
- D1, D2, D3:: first, second and third diagonal branches
- CFX:: circumflex artery
- M1, M2, M3:: first, second and third marginal branches of the circumflex artery
- PL:: postero-lateral branches.

Figure 1 also shows, by way of example, two possible methods of angioplasty intervention by means of stents implemented, respectively:
- in the proximal portion of the right coronary artery CD, and/or
- in the common trunk TC of the left coronary artery.

It is quite clear that the methods of intervention shown are purely examples since the treatment may also relate merely to one of the sites shown and/or (also) to other sites.

It has also been assumed, purely by way of example, that the extent of the lesion treated (and hence the length of the stents used) is such as also to involve some bifurcations such as the branching-off of the conus branch C and of the atrial sinus branch SA (in the case of the right coronary artery) as well as of the anterior interventricular artery IVA (in the case of the left coronary artery).

For simplicity of illustration, it has been assumed that the intervention is directed primarily towards angioplasty treatment solely of the main vessel (the right and/or the left coronary artery). The main object of the solution according to the invention is thus represented, in this case, by the prevention of undesired even partial blocking of the ostium of the branch vessel. It will, however, be appreciated that the solution according to the invention is also ideally suitable for an angioplasty intervention also involving the branch vessel - in accordance with the methods described in the introductory portion of the description.

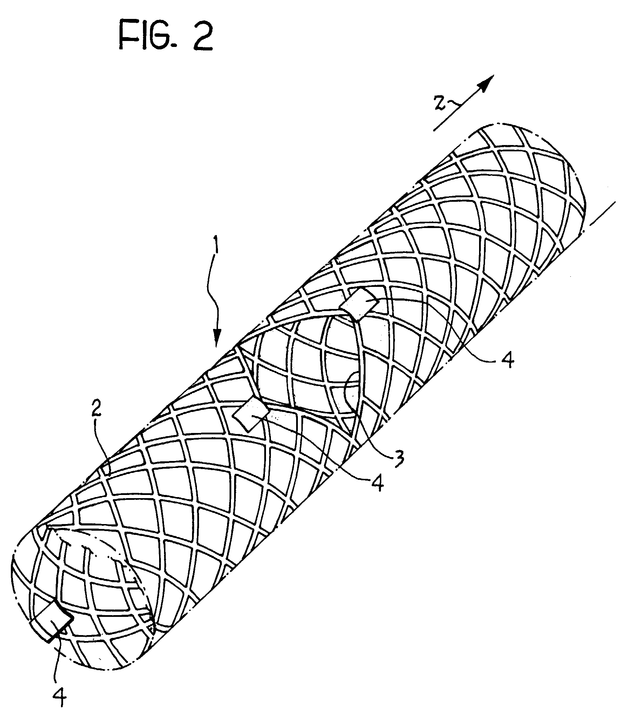

In summary (and with reference again to the documentation mentioned in the introductory portion of the description for a general identification of the methods of use and of the structural features), the stent 1, as shown in Figure 2, is usually produced in the form of a body with a tubular envelope having an overall length of a few millimetres. The body has a wall thickness (the wall usually being of a mesh or loop structure with openings, as will be explained further below) of the order, for example, of approximately 70-140 microns, in view of its possible implantation in a site in which a stenosis is to be remedied. The stent is normally located in situ by catheterization with subsequent radial expansion from an insertion diameter of the order, for example of approximately 1-1.6 mm to an expanded diameter, for example, of the order of approximately 3-5 mm so that, in the expanded condition, the stent supports the lumen, preventing recurrence of a stenosis. In general, the outside diameter in the radially contracted condition is selected so as to allow the stent to be introduced into the lumen being treated, whereas the expanded diameter corresponds, in general, to the diameter to be maintained and established in the lumen once the stenosis has been eliminated.

The stent 1 shown in Figure 2 is shown in the radially expanded condition since, in this condition it is easier to show the characteristics described and claimed.

In order to spread out the stent in situ, the solution most generally used provides for the use of a balloon catheter, the stent being disposed around the balloon of the catheter in the contracted condition and the balloon then being expanded once the stent has been brought to the site in which it is to be positioned. However, other solutions are possible, for example, the use of superelastic materials which cause the stent to expand once the restraining elements which are intended to keep the stent in the contracted condition until the implant site has been reached, are removed. In addition or alternatively, materials having a so-called "shape memory" may also be used to form the stent so as to achieve the radial expansion in the implant position.

Usually (for more precise indications, reference should be made to the bibliographical and patent documentation cited in the introduction to the description), the stent is made of metal which can reconcile two basic requirements of the application, that is, plastic deformability during the expansion stage and the ability to withstand any stresses which would tend to cause the stent to close up again, preserving the expanded shape.

In any case, these technological aspects will not be dealt with in detail in the present description since they are not relevant *per se* for the purposes of understanding and implementing the invention. This also applies basically to the technology for the production of the stents according to the invention. As already stated, in general terms, these adopt the appearance of bodies with tubular envelopes having walls with openings. With regard to the production methods, according to the prior art, at least three basic solutions may be used, that is:
- producing the stent from a continuous tubular blank to be cut up into individual stents, the portions with openings being formed by techniques such as laser-cutting, photo-engraving, electron-discharge machining, etc;
- producing the stent from a strip-like body in which the regions with openings are formed, for example, by the techniques mentioned above, with a view to the subsequent closure of the strip-like element to form a tube, and
- producing the stent from shaped metal wire with subsequent connection of the loops of wire, for example, by micro-welding, brazing, gluing, crimping, etc.

The first solution described is that which is currently preferred by the Applicant for producing the stents according to the embodiments described below.

In any case, this production aspect is not relevant *per se* for the purposes of the implementation of the invention. This also applies with regard to the selection of the individual techniques and of the order in which the various steps described (the production of the portions with openings, parting, any bending of the strip-like element, etc.) are carried out.

The body of the stent 1 extends in a longitudinal direction generally indicated by an axis z. For simplicity of illustration, reference is made herein, purely by way of non-limiting example, to a stent 1, the wall of which has a generally rhombic mesh structure. It will, however, be appreciated that the invention may also be implemented in stents 1 having any wall geometry such as, for example, the geometries described in the previous patent applications relating to angioplasty stents filed by the Applicant. This applies, in particular but not exclusively, to the mesh-like geometry described in European patent application No. 98107382.8.

The main characteristic of the stent 1 according to the invention is that it has a wall structure 2 which has openings and which is generally uniform, with the sole exception of a larger opening 3 shown purely by way of example herein as being located approximately half way along the overall length of the stent 1.

The reference to a structure 2 with openings which is generally uniform (except for the presence of the opening 3) does not exclude the possibility of the body of the stent 1 having differentiated mechanical characteristics, for example, owing to the presence of a segment situated in an end position or substantially in an end position and having a markedly greater resistance than the rest of the stent to radial compression from the radially expanded condition. This characteristic is the subject of a patent application filed by the Applicant on the same date.

What is important for the purposes of the invention is the presence of one or possibly several openings of larger dimensions, such as the opening 3, which are disposed at different points along the stent 1.

The main characteristic of the solution according to the invention is that the opening 3 (for convenience, the presence of only one such opening will be referred to below) is such as to involve - in the region of the longitudinal portion of the stent 1 affected thereby - only a portion of the angular extent of the stent; for example, this may be an angular portion corresponding, for example, and preferably, to an angle of about 60°-75°, and hence less than about 90° (usually with reference to the stent in the radially expanded condition).

This means that, in contrast, for example, with the solution of the prior art specifically cited in the introductory portion of the present description, the opening 3 (which is in fact comparable to an opening markedly larger than the other openings in the wall of the stent) does not involve the whole angular extent of the portion of the wall in which it is disposed.

During implantation, the stent 1 can thus be positioned as shown schematically in Figure 1, that is, by arranging for the opening 3 to be disposed precisely in the region of the ostium of the branch vessel.

On the one hand, this method of operation prevents the occurrence of any of the blockage phenomena mentioned in the introductory portion of the present description and, on the other hand, this method enables a further stent optionally to be introduced through the stent 1 thus positioned, and to be advanced through the opening 3 into the branch vessel in order then to be expanded, achieving full support of the vessels in the region of the bifurcation.

The solution according to the invention ensures a firm and continuous support of the vessel wall even in the region of the bifurcation. The stent wall has a sufficiently dense mesh structure in all of the regions in which it is necessary to perform a supporting action on the vessel wall. The only region in which the structure is lacking is the region of the opening 3. The latter, however, is intended to be positioned in the region of the ostium of the branch vessel where, on the one hand, the occurrence of blockage phenomena is to be prevented and, on the other hand, the problem of providing any supporting action for the wall of a vessel does not arise.

Naturally, moreover, the specific embodiment shown in Figure 2 is given by way of example and is thus related to the generally rhombic mesh structure which is considered specifically herein primarily for simplicity of illustration. According to the geometry of the meshes of the wall of the stent 1, it is thus possible to give the outline of the opening 3 the desired profile, for example, a slightly lobed profile, or a profile which, when the stent 1 is in the radially expanded condition, adopts a substantially circular or slightly squashed outline ideally adapted to the anatomy of the site treated.

Effective use of the solution according to the invention presupposes that, during the implantation stage, it is possible to position the stent 1 precisely both longitudinally (in other words so that the portion of the stent 1 in which the opening 3 is disposed is positioned precisely at the level of the branch vessel) and with regard to its orientation about its principal axis, ensuring that the opening 3 is actually facing the mouth of the branch vessel and not, for example, in a misaligned or even opposed position. This result can be achieved, for example, by a suitable arrangement of references (so-called markers) schematically indicated 4 and formed in accordance with known techniques. These are usually radio-opaque markers present on the body of the stent 1 (for example, also on the outline of the opening 3) such as to enable the person performing the implantation by catheterization to detect and monitor the position and orientation of the stent 1 at the implant site in real time.

Naturally, the principle of the invention remaining the same, the details of construction and forms of embodiment may be varied widely with respect to those described and illustrated without thereby departing from the scope of the present invention.

## Claims

1. An angioplasty stent comprising a body (1) with a wall having a generally tubular envelope with openings, the stent being expandable from a radially contracted condition to a radially expanded condition, characterized in that:
- the body has a configuration (2) with openings, the configuraiton of the body being substantially uniform except for at least one opening (3) having dimensions substantially larger than the dimensions of the remaining openings provided in the wall, the at least one opening (3) being located in a respective longitudinal portion of the body (2) of the stent and affecting only a portion of the angular extent of the said portion of the body of the stent, and
- marker means (4) are provided for detecting the angular position of the stent (1) at the implant site.

2. A stent according to Claim 1, characterized in that the at least one opening (3) affects a portion of the angular extent of the said portion of the body of the stent no greater than approximately 90° when the stent is in the radially expanded condition.

3. A stent according to Claim 2, characterized in that the at least one opening (3) affects a portion equal to approximately 60°-75° of the angular extent of the said portion of the body of the stent when the stent is in the radially expanded condition.
